Europäisches Patentamt

European Patent Office

Office européen des brevets

(19)

(11) Veröffentlichungsnummer: **0 018 628**
**B1**

(12) **EUROPÄISCHE PATENTSCHRIFT**

(45) Veröffentlichungstag der Patentschrift: **11.04.84**

(51) Int. Cl.³: **C 12 Q 1/48**

(21) Anmeldenummer: **80102321.9**

(22) Anmeldetag: **30.04.80**

(54) Verfahren zur Bestimmung der Gamma-Glutamyltranspeptidase.

(30) Priorität: **04.05.79 DE 2917999**

(43) Veröffentlichungstag der Anmeldung:
**12.11.80 Patentblatt 80/23**

(45) Bekanntmachung des Hinweises auf die Patenterteilung:
**11.04.84 Patentblatt 84/15**

(84) Benannte Vertragsstaaten:
**AT BE CH DE FR GB IT LI NL SE**

(56) Entgegenhaltungen:
**DE - A - 2 333 798**
**DE - B - 2 259 512**
**FR - A - 2 071 603**
**FR - A - 2 106 163**

**CHEMICAL ABSTRACTS, Band 90, no. 9, 26**
**Februar 1979, Zusammenfassung 68524n, Seite**
**207 Columbus, Ohio, US**
**CHEMICAL ABSTRACTS, Band 86, no. 8, 21**
**Februar 1977, Zusammenfassung 47294q, Seite**
**239**

(73) Patentinhaber: **BEHRINGWERKE**
**AKTIENGESELLSCHAFT**
**Postfach 1140**
**D-3550 Marburg/Lahn (DE)**

(72) Erfinder: **Heber, Helmut, Dr.**
**Am Ziegenberg 8**
**D-3550 Marburg/Lahn (DE)**
Erfinder: **Kohl, Helmut, Dr.**
**Goethe-Strasse 32**
**D-3552 Wetter (DE)**

(74) Vertreter: **Meyer-Dulheuer, Karl-Hermann, Dr.**
**et al,**
**HOECHST Aktiengesellschaft Zentrale**
**Patentabteilung Postfach 80 03 20**
**D-6230 Frankfurt/Main 80 (DE)**

# 0 018 628

## Verfahren zur Bestimmung der $\gamma$-Glutamyltranspeptidase

Die Erfindung betrifft ein Verfahren zur Bestimmung der $\gamma$-Glutamyltranspeptidase in wässrigen Systemen, vor allem in biologischen Flüssigkeiten, insbesondere in Blutserum. Es findet hauptsächlich Verwendund zur Erkennung und Verlaufsdiagnose von Lebererkrankungen.

Die Bestimmung der $\gamma$-Glutamyltranspeptidase ($\gamma$-GT)-Aktivität erfolgt nach dem bekannten Reaktionsschema:

$$\gamma\text{-Glutamyl-p-Nitranilid} + \text{Glycylglycin} \rightleftharpoons \text{p-Nitranilin} + \text{Glutamylglycylglycin}$$

Die Freisetzung des gelbgefärbten p-Nitranilins kann optisch verfolgt werden. Sie ist ein direktes Maß für die vorhandene $\gamma$-GT-Aktivität.

Der Vorzug des einfachen Nachweises des Nitranilins ist z. Zt. mit dem Nachteil verknüpft, daß das Substrat $\gamma$-Glutamyl-p-Nitranilid in wässrigen Lösungen mit pH-Werten, wie sie bei der Bestimmung der $\gamma$-GT eingehalten werden müssen, sehr schlecht löslich ist. Dies ist u.a. aus den Patentanmeldungen DE—A—20 42 829 und DE—A—22 59 512 bekannt. Man hat schon mehrfach versucht, dieses Problem zu lösen. So wurde das Substrat beispielsweise in verdünnter Salzsäure oder in organischen Lösungsmitteln in hoher Konzentration vorgelöst und in dieser Form den Bestimmungsansätzen zugefüft, wodurch eine starke Verschiebung des pH-Wertes vermieden wird. Zu dem Nachteil dieser wenig eleganten Methode kommt noch die Instabilität des Substrates hinzu, die in stark saurem, wässrigen Milieu beobachtet wird. Daher sind solche Lösungen nur kurzfristig für Messungen einsetzbar.

Gemäß einem anderen bekannten Verfahren wird das $\gamma$-Glutamyl-p-Nitranilid bei einer Temperatur zwischen 50 und 60°C aufgelöst und danach auf Zimmertemperatur abgekühlt. Auch hierbei besteht die Gefahr der spontanen Hydrolyse der chromophoren Gruppe. Weiterhin ist das Auflöseverfahren zeitraubend und häufig wegen der Rekristallisation des Substrates ohnehin nicht erfolgereich.

Weiterhin ist aus Chemical Abstracts Band 90, 1979, Seite 207, 68524n, ein Verfahren bekannt, das im wesentlichen darin besteht, $\gamma$-Glutamyl-p-Nitranilid in Salzsäure zu lösen, auf Papier aufzubringen, zu trocknen, das imprägnierte Papier mit einem Puffer zu tränken und damit $\gamma$-Glutamyl-transpeptidase nachzuweisen.

In der französischen Patentschrift 2 071 603 ist ein Reagenz zur Bestimmung der $\gamma$-Glutamyltranspeptidase beschrieben, das zus $\gamma$-Glutamyl-p-Nitranilid, einem oder mehreren oberflächenaktiven amphoteren oder nichtionischen Agentien und einer Kollidin-, Borsäure-, Diethylbarbitursäure- oder Ammoniumpufferlösung besteht.

Diese Methoden sind für die routinemäßige Anwendung des Testes in klinischen Laboratorien unbefriedigend, so daß die Aufgabe bestand, die damit verbundenen Nachteile zu beseitigen. Das Substrat $\gamma$-Glutamyl-p-Nitranilid soll bei der für die Durchführung der Bestimmung einzuhaltenden Temperatur von etwa 25°C ausreichend gut löslich sein.

Es wurde nun befunden, daß sich trockenes $\gamma$-Glutamyl-p-Nitranilid, vorzugsweise als Lyophilisat, im Testpuffer bei 20 bis 25°C nahezu augenblicklich auflöst, wenn das Substrat protonisèrt, das heißt als Salz vorliegt. Dies bedeutet, daß das $\gamma$-Glutamyl-p-Nitranilid vor der Trocknung mit einer Säure im stöchiometrischen Verhältnis oder im Überschuß zu den Basenäquivalenten versetzt wird.

Gegenstand der Erfindung ist deshalb ein Verfahren zur quantitativen Bestimmung von $\gamma$-Glutamyltranspeptidase unter Verwendung eines lyophilisierten Salzes von $\gamma$-Glutamyl-p-Nitranilid mit einer Säure, dadurch gekennzeichnet, daß man dieses Salz in einem Puffer löst, mit der $\gamma$-Glutamyltranspeptidase-haltigen Probe versetzt und die Menge des freigesetzten p-Nitranilids bestimmt.

Da zudem gefunden wurde, daß das Salz des $\gamma$-Glutamyl-Nitranilids nach Wiederauflösung eine bessere Farbstabilität besitzt, wenn es mit einem quaternären Polykation auf Basis von Polyvinylpyrrolidon versetzt wird, gehört es zum Gegenstand der Erfindung, daß die trockene Zubereitung gegebenenfalls mit einem solchen hochmolekularen Lösungsstabilisator versetzt wird.

Im engeren Sinne gehört est zum Gegenstand der Erfindung, daß als Substrat für die Bestimmung von $\gamma$-Glutamyltranspeptidase die Ammoniumsalze des $\gamma$-Glutamyl-p-Nitranilids gegenbenenfalls mit einem Überschuß an Säure in dem beanspruchten Verfahren verwendet werden.

Als Säuren, die hierfür in Frage kommen, sind praktisch alle organischen oder anorganischen Säuren zu nennen, die ein Ammoniumsalz des $\gamma$-Glutamyl-p-Nitranilids zu bilden vermögen, z.B. Schwefelsäure, die Phosphorsäuren, alle Halogenwasserstoff-Säuren, Oxyhalogenwasserstoff-Säuren, Essigsäure und Trifluoressigsäure. Bewährt haben sich Kombinationen aus einer anorganischen und einer organischen Säure wie Di-, Tri-, Tetra-Carbonsäuren beziehungsweise-Hydroxycarbonsäuren, beispielsweise Pimelinsäure, Maleinsäure, Malonsäure, Bernsteinsäure, Glutarsäure, Adipinsäure, Fumarsäure, Äpfelsäuren, Weinsäuren, Citronensäure, Ascorbinsäure, Glucuronsäure. Vorzugsweise wird eine anorganische Säure, z.B. Salzsäure, in Kombination mit einer organischen Säure, z.B. Weinsäure, verwendet. Bezogen auf ein Basenäquivalent des Nitranilids ist das Verhältnis von etwa 0.9

2

**0 018 628**

Aquivalenten der anorganischen Säure und etwa 0,1 Aquivalenten der organischen Säure besonders günstig.

Zur Herstellung des Substrates eignen sich alle gängigen Methoden der organischen Chemie zur Herstellung von Ammoniumsalzen von primären Aminen. Ein Überschuß der Säuren ist so lange nicht schädlich, so lange das Substrat ohne Nachteile gefriergetrocknet werden kann und das wiederaufgelöste Substrat die Kapazität des Testpuffers nicht über schreitet. Am einfachsten geht man von einer wässrigen Lösung des $\gamma$-Glutamyl-p-Nitranilids aus, stellt, das stöchiometrische Verhältnis des Anilids zu einer Säure fest, versetzt die Lösung mit'der betreffenden Säure in dem gewünschten Verhältnis, gegebenenfalls mit einem Überschuß von ca. 20% und trocknet das Produkt.

Ein anderer sehr einfacher Weg zur Herstellung des Substrats ist, die wässrige Lösung des Nitranilids mit der gewünschten Säure anzusäuren, wonach die Trockung durchgeführt werden kann.

Der wesentliche Vorteil des Trockenproduktes besteht darin, daß es hierbei zu keiner nennenswerten Hydrolyse vor dessen Verwendung im Test kommt und demgemäß eine Störung durch einen Leerwert hoher optischer Extinktion ausbleibt.

., Eine Verbesserung der Substrateigenschaf

Eine Verbesserung der Substrateigenschaft wird, wie bereits erwähnt, dadurch erreicht, daß dem Substrat ein quaternäres Polykation auf Basis von Polyvinylpyrrolidin in einer Konzentration von 0,1—100 mg/ml Testlösung zugesetzt wird. Dieser Befund ist überraschend, da es bekannt ist, daß übliche kationische bzw. anionische Detergenzien die $\gamma$-Glutamyltranspeptidase-Aktivität hemmen bzw. vermindern.

Demgegenüber beeinflußen quaternäre Polykationen auf Basis von Polyvinylpyrrolidon die Enzymaktivität nicht meßbar. Den gleichen Effekt zeigen äthoxylierte Alkylphenole oder äthoxylierte Phenole sowie Tetrafluoräthylen-Polymerisate mit verzweigten Perfluorgruppen. Diese Polymeren verzögern einzeln oder in Kombination die Rekristallisation des $\gamma$-Glutamyl-p-Nitranilids bei Raumtemperatur.

Das gegebenenfalls mit den Polymeren versetzte Salz des $\gamma$-Glutamyl-p-Nitranilids wird in fester Form zur Verfügung gehalten. Dies kann in Trockenabfüllungen oder in Tabletten verpreßt, geschehen.

Weitere Verbesserungen hinsichtlich der Stabilität des Produktes werden erreicht durch übliche Zuschläge bei der Gefriertrocknung von Produkten wie Gelatine oder Gelatine-Hydrolysaten, Zuckeralkoholen oder Kohlenhydraten, Pufferkomponenten, Konservierungsmitteln und/oder Lösungsvermittlern. In der bevorzugten Form wird Mannit verwendet in einen Konzentrationsbereich von 1—50 mg/ml.

Auch das in die Reaktion der Transpeptidase eingehende Glycylglycin kann in der trockenen Zubereitung bereits enthalten sein, so daß für den Test lediglich das Substratgemisch in Wasser aufgelöst werden muß, wonach das Enzym bestimmt werden kann.

Ein weiterer Vorteil des erfindungsgemäßen Verfahrens besteht darin, daß es auch auf solchen üblichen Analysenautomaten ausgeführt werden kann, auf denen eine 3-Stufen-Testdurchführung nicht möglich ist. Im beanspruchten Verfahren kann das Salz des Glutamyl-Nitranilids in einem Puffer zu einer Vorratslösung gelöst werden, von der eine bestimmte Menge zu einer bestimmten Probenmenge gegegen wird, so daß lediglich zwei Pipettierschritte auszuführen sind.

Das folgende Beispiel soll die Erfindung näher erläutern.

Beipiel

Das Substrat wird wie folgt hergestellt:

a) Substratkomponenten
1) 1,17 g $\gamma$-Glutamyl-p-nitranilid (1,176 g/1000 ml=4,4 mM)
2) 8,7 g Glycylglycin
3) 16 g Mannit
4) 10 g Kalium-Natrium Tartrat . 4$H_2$
5) 40 g Quarternäres Polyamin
6) 2 n HCl bis pH 2—3
7) dest. Wasser→1 Literf

Die Komponenten 1—5 werden in ca. 600 bis 700 ml dest. Wasser aufgenommen, auf etwa 40°C erwärmt und mit der Komponenten 6 bis zu einem pH-Wert zwischen 3 und 4 versetzt. Nach vollständiger Auflösung aller Substratkomponenten wird die Lösung auf etwa 20°C abgekühlt und der pH-Wert mit 2 n HCl (6) auf 2 bis 3 eingestellt. Die Lösung wird schließlich mit destilliertem Wasser (7) auf 1,0 Liter aufgefüllt.

Es hat sich als zweckmäßig erwiesen, die Bestimmung der $\gamma$-Glutamyltranspeptidase mit 2 ml dieser Substratlösung durchzuführen, so daß man hierfür Abfüllungen von 2 ml herstellt und anschließend gefriertrocknet. Für die Durchführung des Testes wird das gefriergetrocknete Produkt mit der Pufferlösung b) wiederaufgelöst.

3

**0 018 628**

b) Pufferlösung
    8) 24 g Tris-(hydroxymethyl)-aminomethan
    9) 5 g Natriumazid werden in etwa 800 ml dest. Wassers gelöst, mit 2 n HCl auf pH 9,2 bis 9,5 eingestellt und mit dest. Wasser auf 1,0 Liter aufgefüllt, Wird das lyophilisierte Substratgemisch nach a) mit 2 ml Pufferlösung der erwähnten Zusammensetzung versetzt, stellt sich ein pH-Wert von etwa 8,2 ein.

## Patentansprüche

1. Verfahren zur quantitativen Bestimmung von gamma-Glutamyltranspeptidase unter Verwendung eines lyophilisierten Salzes von gamma-Glutamyl-p-Nitranilid mit einer Säure, dadurch gekennzeichnet, daß man dieses Salz in einem Pufferlöst, mit der gamma-Glutamyltranspeptidase-haltigen Probe versetzt und die Menge des freigesetzten p-Nitranilins bestimmt.

2. Verfahren nach Anspruch 1, dadurch gekennzeichnet, daß man das Salz in einem Puffer löst, der als Lösungsstabilisator ein quaternäres Polykation auf Basis von Polyvinylpyrrolidon enthält.

## Revendications

1. Procédé de détermination quantitative de la gamma-glutamyltranspeptidase en utilisant un sel lyophilisé du gamma-glutamyl-p-nitranilide avec un acide, caractérisé en ce qu'on dissout ce sel dans un tampon, on l'additionne de l'échantillon contenant de la gamma-glutamyltranspeptidase et on détermine la quantité de p-nitraniline libérée.

2. Procédé suivant la revendication 1, caractérisé en ce qu'on dissout le sel dans un tampon qui contient comme stabilisant de la solution un polycation quaternaire à base de polyvinylpyrrolidone.

## Claims

1. Process for quantitatively determining gamma-glutamyltranspeptidase by means of a lyophilized salt of gamma-glutamyl-p-nitranilide with an acid, which process comprises dissolving that salt in a buffer, adding the sample containing the gamma-glutamyltranspeptidase and determining the amount of p-nitraniline set free.

2. Process according to claim 1 comprising dissolving the salt in a buffer which contains a quarternary polycation derived from polypyrrolidone.

4